# EUROPÄISCHE PATENTSCHRIFT

(11) **EP 0 561 821 B1**
(45) Veröffentlichungstag und Bekanntmachung des Hinweises auf die Patenterteilung: **28.12.1994**
(21) Anmeldenummer: 91920426.3
(22) Anmeldetag: 21.11.1991
(51) Int. Cl.: B01J 13/16, A61K 9/50

(54) **VERFAHREN ZUR HERSTELLUNG VON KOLLAGENPARTIKELN UND IHRE VERWENDUNG ALS WIRKSTOFFTRÄGER**
METHOD OF PRODUCING COLLAGEN PARTICLES, AND THE USE OF SUCH PARTICLES AS SUBSTRATES FOR ACTIVE SUBSTANCES
PROCEDE D'OBTENTION DE PARTICULES DE COLLAGENE ET LEUR UTILISATION COMME SUPPORT DE MATIERE ACTIVE

(30) Priorität: 06.12.1990 DE 4038887
(43) Veröffentlichungstag der Anmeldung: 29.09.1993
(73) Patentinhaber: LTS Lohmann Therapie-Systeme GmbH & Co. KG, 56513 Neuwied (DE)
(72) Erfinder: KREUTER, Jörg, D-6380 Bad Homburg (DE); SCHERER, Dieter, D-6454 Bruchköbel (DE); MÜLLER, Walter, D-5450 Neuwied (DE); ROREGER, Michael, D-5450 Neuwied 22 (DE)
(74) Vertreter: Flaccus, Rolf-Dieter, Dr.
(86) Internationale Anmeldenummer: EP9102195
(87) Internationale Veröffentlichungsnummer: WO9210287

(56) Entgegenhaltungen:
- EP-A- 0 213 303
- US-A- 4 798 786
- Derwent Publications LTD, London, GB, Database WPIL,Nr. 85-260393
- Derwent Publications LTD, London, GB, Database WPIL, Nr. 85-120200E20R

## Beschreibung

Die Erfindung betrifft ein Verfahren zur Herstellung von Kollagenpartikeln, deren Größe im Mikro- und Nanometerbereich liegt, und ihre Verwendung als Wirkstoffträger in Medizin, Nahrungsmittelindustrie und Kosmetik.

Der Einschluß zahlreicher Wirk- und Inhaltsstoffe, wie Farbstoffe, Katalysatoren, Enzyme, radioaktive Stoffe, Aromastoffe, flüchtige Verbindungen und ähnliche in ebenso zahlreichen Hüllmaterialien in Kapseln einer Größe im Mikro- und Nanometerbereich ist bekannt.
Auf pharmazeutischem Gebiet finden z. B. ätherische Öle in Gelatinehüllen als Aromaträger Verwendung, die das Aroma erst nach Auflösen der Hülle freigeben.
Zweck der Verkapselung ist neben der Freigabe des Inhaltsstoffes nach Auflösen der Hülle z. B. der Schutz von Wirkstoffen gegen Sauerstoff, Feuchtigkeit oder andere chemische Agentien, die Überführung einer Flüssigkeit in ein "trockenes" Pulver, die Herstellung von Retardformen von Wirkstoffen durch entsprechende Behandlung oder Auswahl des Hüllmaterials oder die Herstellung von Wirkstoff-Hilfsstoff-Komplexen durch Oberflächenbeladung von Trägerpartikeln mit Wirkstoffen.
Partikelgrößen im Nanometerbereich bieten darüber hinaus die Möglichkeit, diese infolge der extrem kleinen Abmessungen der Kapseln mitsamt dem eingeschlossenen oder adsorbierten aktiven Material in Wasser kolloidal zu lösen. Dadurch ist es möglich, solche Lösungen auch intravenös zu verabreichen.

Kollagen ist der Hauptbestandteil des Bindegewebes bei Wirbeltieren und ist nahezu ubiquitär.
Partikuläre Arzneimittelträger aus Kollagen haben gegenüber anderen Trägersystemen den Vorteil, daß aufgrund der Biokompatibilität und des vollständigen Bioabbaus und Stoffwechselumsatzes der Partikel toxikologische Probleme bei lokaler und systemischer Anwendung nicht zu erwarten sind.

Die Verwendung von Kollagen als Kapselhüllmaterial bietet darüber hinaus den Vorteil, daß bei Substanzen mit hoher Eiweißaffinität, die sehr gut an Proteinpartikel zu binden sind, eine hohe Beladung des Trägersystems möglich ist.
Die Kapazität zur adsorptiven Beladung des Trägersystems liegt in diesen Fällen bei gleicher Partikeloberfläche um ein Vielfaches über der von beispielsweise Trägerpartikeln auf Acrylatbasis.

Verfahren zur Herstellung von Kollagenmikropartikeln sind beispielsweise in der US-PS 4 565 580 und in der US-PS 4 107 288 beschrieben. Die Bezeichnung "Mikropartikel" wird in dem Sinne gebraucht, daß hierdurch Partikelgrößen im Mikrometerbereich und kleiner bezeichnet sind.
Gemeinsames Merkmal der dort beschriebenen Verfahren ist, daß die Mikropartikel nach dem Prinzip der Koazervation hergestellt werden.
Bei diesem Verfahren werden einer molekulardispersen Lösung von Kollagen desolvatierende Agentien, wie z. B. Alkohole oder Elektrolyte zugesetzt.
Durch den Zusatz wird die Löslichkeit des Proteins vermindert, was zu einer Trübung der Lösung führt die das Entstehen lichtstreuender Proteinaggregate anzeigt.

Durch Abtrennen des Koazervates und Trocknen erhält man letztlich Mikrokapseln, die durch nachträgliche Behandlung gehärtet werden können. Die Partikeldurchmesser liegen im Bereich von 10 - 1000 µm (US-PS 4 565 580) bzw. von 10 - 1000 nm (US-PS 4 107 288).
Im US-PS 4 837 285 wird ein Verfahren beschrieben, bei dem eine wäßrige Kollagendispersion durch eine vibrierende Hohlröhre definierten Durchmessers gedrückt wird. Am Ende des Röhrchens bilden sich Tröpfchen, die in ein Kältebad aus flüssigen Stickstoff tropfen. Nach dem Verdampfen des Stickstoffs werden die gefrorenen Tröpfchen lyophilisiert und anschließend in wäßriger Dispersion zwecks Nachhärtung vernetzt. Die Partikeldurchmesser liegen zwischen 100 und 400 µm.

Die genannten, nach Verfahren des Standes der Technik erhaltenen Größenerteilungen der Kollagenmikropartikel zeigen bereits die gravierenden Nachteile.
Zum einen streut die Größenverteilung der Partikel über einen sehr breiten Bereich. Homogene Partikelfraktionen mit sehr enger Partikelverteilung können nach diesem Verfahren nicht erhalten werden, da Zusammenlagerung der Kollagenmoleküle und die Partikelagglomeration zufällig erfolgen und kaum zu steuern sind.
Insbesondere lasen sich nach diesen Verfahren keine definierten Kollagenpartikelfraktionen mit einem Durchmesser im unteren Mikrometer- und im Nanometerbereich gewinnen, so daß die nach diesem Verfahren hergestellten Mikropartikel für Zubereitungen zur intravenösen Injektion nicht geeignet sind.

Die US-A-4 798 786 beschreibt die Herstellung von nachgiebigfesten Mikropartikeln für den Einschluß lebender Zellen. Die bekannte Erfindung beruht auf der Ermittlung spezieller Bedingungen zur Herstellung von Mikrokapseln mit durchlässigen Wänden von kontrollierbarer Porosität. Hierfür werden die Kapselwände mit einem Schwächungs-Enzym so lange behandelt, bis eine zufriedenstellende Erweiterung der Porengroße in den Wänden der Kapseln erreicht ist. Ein solches Verfahren ist jedoch für die als Wirkstoffträger verwendeten Mikrokapseln wegen der Durchlässigkeit der porösen Wände ungeeignet.

Der vorliegenden Erfindung liegt die Aufgabe zugrunde, ein Verfahren zur Herstellung von homogenen Kollagenpartikeln im Mikro- und Nanometer-Bereich mit einer durch das Verfahren zu steuernden engen Partikelgrößenverteilung zu finden, und zwar für eine geeignete Verwendung als Wirkstoffträger in Medizin-, Nahrungsmittelindustrie und Kosmetik.

Die Aufgabe wird bei einem Verfahren zur Herstellung von vernetzten Kollagenmikropartikeln im Mikro- und Nanometerbereich, wobei eine Dispersion oder Losung von Kollagen in Wasser unter Zugabe von Emulagatoren in einer nicht wassermischbaren organischen Phase unter Ausbildung einer W-O-Emulsion fein in diskreten Tröpchen verteilt wird, und anschließend unter Zugabe eines Vernetzungsmittels das Kollagen in der Tröpfchen-Grenzfläche vernetzt wird, und schließlich die so hergestellten Kollagenpartikel durch Abtrennen der organischen Phase und durch Waschen gereinigt und isoliert werden, mit der Erfindung in überraschenderweise dadurch gelöst, daß die Kollagenpartikel nach Isolierung und Reinigung als Wirkstoffträger für Medizin, Kosmetik oder den Nahrungsmittelbereich mit Wirkstoffen beladen werden.

Weiterhin ist vorgesehen, daß die Kollagenpartikel nach Herstellung, Abtrennung und Reinigung mit einer hohen Temperaturerniedrigungsrate (Gradienten) auf Temperaturen ≦-50°C unter Bildung möglichst kleiner Eiskristalle schockgefroren, anschließend unter Vakuum gefriergetrocknet, sodann zur Wirkstoffbehandlung in Wasser redispergiert, der Dispersion Wirkstoff, Wirkstofflösung bzw. -dispersion zugesetzt und die adsorptiv mit Wirkstoff beladenen Partikel abgetrennt und gereinigt werden.

Zur Herstellung der Partikel wird aus einer wäßrigen Kollagendispersion, die natives oder mittels chemischer Mittel bzw. physikalischer Einflüsse denaturiertes, aus dem Fachmann bekannten Quellen unterschiedlicher Herkunft isoliertes Kollagen enthalten kann, einem Emulgator und einer organischen Phase auf dem Fachmann bekannte Weise eine Emulsion hergestellt. Die Bestimmung des Emulsionstyps kann über Leitfähigkeitsmessung erfolgen.

Emulgatoren, die bei den erfindungsgemäßen Verfahren vorteilhaft einsetzbar sind, sind Polyethylenglykolfettsäureester, wie z. B. Polyethylenglykol-400-stearat, Polyethylenglykolfettalkoholether, wie z. B. Polyethylenglykol-200-laurylether, Polyethylenglykol-sorbitan-fettsäureester, wie z . B. Polyethylenglykol-(20)-sorbitanmonooleat, Partialfettsäurester mehrwertiger Alkohole, monooleat, Partialfettsäurester mehrwertiger Alkohole, wie z. B. Glycerinmonostearat oder Sorbitantrioleat, oder Partialfettsäureester von Zuckern, wie z. B. Saccharosemonolaurinsäureester.

Die nicht-wassermischbare organische Phase kann natürliche, halbsynthetische oder synthetische flüssige Fette, öle oder Wachse enthalten, wie z. B. Olivenöl, Rizinusöl, Baumwollsaatöl, Sojaöl, hydriertes Erdnußöl, Triglyceridgemische (Miglyol®, Softisan®), Silikonöl, Ölsäureoleylester, Isopropylmyristat oder Ethyloleat.

Die Herstellung der Emulsion ist der entscheidende Schritt zur Beeinflußung von Größe und Homogenität der letztlich resultierenden Mikropartikel. Durch Art des Emulgators bzw. des Emulgatorkomplexes sowie durch die Art und Intensität des Emulgierverfahrens wird die Größe der dispergierten Wassertröpfchen in der organischen Phase bestimmt und gesteuert.

Die Kollagenmoleküle werden dann durch Zugabe eines Vernetzungsmittels in der Tröpfchengrenzfläche polymerisiert. Als Vernetzungsmittel können mono- oder bifunktionelle Aldehyde, wie z. B. Formaldehyd, Glutardialdehyd oder Dialdehydstärke, oder bifunktionelle Isocyanate, wie z. B. Hexamethylendiisocyanat, verwendet werden.

Anschließend wird die organische Phase abgetrennt, und die Partikel werden durch Waschen gereinigt, d. h. von Resten der organischen Phase, des Emulgators und des Vernetzungsmittels befreit.

Um Aggregate zu zerteilen, kann es notwendig sein, die Kollagen-Mikropartikel im Ultraschallbad zu redispergieren. Die Bestimmung der Partikelgröße und der Größenverteilung erfolgt nach Gefriertrocknung und Redispergierung mittels Photonkorrelationsspektrometrie. Dabei wird ein Laserstrahl mit 20 Impulsen pro Sekunde durch eine Küvette geschickt, die mit einer verdünnten, kolloidalen Lösung an Kollagenpartikeln gefüllt ist. Das durch die Partikel gestreute Laserlicht wird im Winkel von 90° zum einstrahlenden Laser über eine Photozelle registriert.

Die Streuung ist abhängig von der Größe der Partikel, und durch Umformung des erhaltenen elektrischen Signals mittels mathematischer Gleichung läßt sich auf die Partikelgröße schließen.

Bei jedem Impuls wird, bedingt durch Brownsche Molekularbewegung, eine andere "Partikel-Population" getroffen. So wird durch die Information, die durch die große Zahl der Impulse (> 2500) entsteht, ein genaues Bild über Durchschnittsgröße und Größenverteilung der Partikel geliefert.

Der Emulsion können vor der Vernetzung der Partikel Wirkstoffe zugesetzt werden.
Die Wirkstoffe werden während der Partikelherstellung durch Grenzflächenpolymerisation in die Partikel eingeschlossen bzw. nach der Partikelbildung an der Oberfläche der Teilchen adsorbiert.
Wenn die Wirkstoffbeladung nach der Partikelherstellung erfolgen soll, so werden die Kollagenpartikel nach Herstellung, Abtrennung und Reinigung einem beliebigen, dem Fachmann bekannten Trocknungsprozeß unterworfen, wobei vorzugsweise das Verfahren der Gefriertrocknung angewandt wird.
Die Partikel werden mit einer hohen Temperaturerniedrigungsrate in einem Lyophilisator auf Temperaturen ≦ - 50° C schockgefroren zur Bildung möglichst kleiner Eiskristalle und anschließend unter Vakuum gefriergetrocknet.
Durch den Trocknungsvorgang wird die Stabilität der Partikel während der Lagerung bis zu ihrer weiteren Verarbeitung erhöht.
Zur nachträglichen Beladung der getrockneten Partikel mit Wirkstoff werden diese zunächst in Wasser redispergiert; der Dispersion wird dann der Wirkstoff, eine Wirkstofflösung oder eine -dispersion zugesetzt.
Die adsorptiv mit Wirkstoff beladenen Partikel werden anschließend abgetrennt und gereinigt.

Nach dem erfindungsgemäßen Verfahren ist die Herstellung von Kollagenpartikeln mittels Emulsionspolymerisation mit sehr kleinem Durchmesser möglich, die in Zubereitungen zur intravenösen Injektion oder zur Anwendung an Auge eingesetzt werden können; das vergleichsweise einfache Verfahren ermöglicht eine genaue Prozeßführung und damit eine bessere Kontrolle der Herstellung homogener Partikelgesamtheiten in der jeweils gewünschten engen Größenverteilung in hoher Ausbeute.

Die Erfindung wird anhand der folgenden Beispiele veranschaulicht :

### Beispiel 1 :

### Herstellung von Kollagenmikropartikeln aus einer W/O-Emulsion :

In 100g Baumwollsaatöl, die in einer Reibschale vorgelegt werden, werden mittels eines Pistills 15g Sorbitanmonolaurat homogen eingearbeitet. Nach 10 Minuten werden 25g einer 1%igen Dispersion von nativem Kalbshautkollagen in fünf Anteilen zugegeben und sorgfältig eingearbeitet. Die Emulsion wird 3 x 10 Minuten im Ultraschallbad unter Kühlung homogenisiert.
In einem weiteren Verfahrensschritt wird die Emulsion über jeweils 3 x 15 Sekunden mit zwischenzeitlicher Kühlung mit einem hochtourigen Homogenisiermischer behandelt. Die Emulsion wird mit 400 rpm auf einem Magnetrührer gerührt, zum Vernetzen der Partikel werden 5,0 ml Glutaraldehydlösung (25%ig) zugesetzt. Die Reaktion wird nach 10 Minuten durch Zugabe von 7,0 ml von Na-Disulfitlösung (25%ig) gestoppt, danach wird die Mischung aufgearbeitet.

Die Zubereitung wird dreimal mit einem Überschuß Ether versetzt und jeweils 10 Minuten bei 2500 rpm zentrifugiert; dieser Vorgang wird noch zweimal mit einem Überschuß Wasser wiederholt. Die Partikel werden entweder gefriergetrocknet oder sofort weiter verarbeitet.

Im vorliegenden Beispiel wurden Kollagenpartikel mit einem durchschnittlichen Durchmesser von 2,15 µm erhalten; 90 % der Partikel waren kleiner als 3,2 µm.
Die Ausbeute an Kollagenpartikeln betrug ca. 95%, bezogen auf die eingesetzte Menge an reinem Kollagen.

### Beispiel 2 :

Die Versuchsdurchführung erfolgte wie in Beispiel 1 beschrieben, wobei anstelle einer 1%igen Dispersion von nativem Kalbshautkollagen eine 1%ige Dispersion von denaturiertem Kalbshautkollagen eingesetzt wurde.
Es wurden Partikel mit einem durchschnittlichen Durchmesser von 320 nm erhalten. Ausbeute : 90 %.

### Beispiel 3 :

### Herstellung von Kollagenmikropartikeln aus einer W/O-Emulsion

10g Polyoxyethylen-(20)-sorbitan-monooleat werden in 25ml einer 0,75%igen Dispersion von nativem Kalbshautkollagen verteilt. Nach 10 Minuten werden 100g Baumwollsaatöl in fünf Teilen zugesetzt und verrührt. Anschließend wird die Emulsion jeweils 5 Minuten mit dem hochtourigen Homogenisiermischer und dem Spalthomogenisator bearbeitet.
Dann wird 1,0 g Glutaraldehydlösung (25%ig) zugesetzt und die Dispersion 10 Minuten gerührt. Um den Überschuß an Glutaraldehyd abzufangen, werden 1,4 ml Na-Disulfitlösung (25%ig) zugesetzt. Die Entfernung der organischen Phase erfolgt wie in Beispiel 1 beschrieben. Ausbeute : 92 %.

Die erhaltenen Partikel wiesen einen durchschnittlichen Durchmesser von 1,23 µm auf. 90% der Partikel waren kleiner als 2,2 µm.

### Beispiel 4 :

### Beladung der Kollagenpartikel mit Ethacridinlactat

50 mg Partikel werden in 50 ml Wasser im Ultraschallbad dispergiert. Zu der Partikeldispersion werden 25 mg Ethacridinlactat zugesetzt und 24 Stunden mit 200 rpm gerührt.
Zur Bestimmung der Beladung wird die Suspension ultrafiltriert, der Arzneistoffgehalt des Überstandes wird bestimmt, und es wird anschließend auf die Partikelbeladung zurückgerechnet. Die Partikelbeladung beträgt 12 mg auf 50 mg Kollagenpartikel.
Die absolute Beladung beträgt somit 24 Gewichtsprozent, bezogen auf die Partikel, und 48 Gewichtsprozent, bezogen auf den eingesetzten Arzneistoff.

### Beispiel 5 :

### Beladung der Kollagenpartikel mit Chlorophyll

250 mg Kollagenpartikel werden in 22,5ml physiologischer NaCl-Lösung dispergiert. Die Dispersion wird 10 Minuten beschallt. Anschließend werden 2,5 ml gesättigte ethanolische Chlorophyll-Lösung zugesetzt und ca. 24 Stunden mit 300 rpm gerührt. Die Partikel werden über eine GPC-Säule gereinigt, die mit Sephadex^{®} G-50 (vernetztes Polysaccharid) gepackt ist.
Es zeigt sich keine zweite Bande; dies deutet darauf hin, daß die Beladung quantitativ erfolgte. Die erhaltenen Partikel werden gefriergetrocknet.

## Patentansprüche

1. Verfahren zur Herstellung von vernetzten Kollagenmikropartikeln im Mikrometer- und Nanometerbereich, wobei eine Dispersion oder Lösung von Kollagen in Wasser unter Zugabe von Emulgatoren in einer nicht wassermischbaren organischen Phase unter Ausbildung einer W/O-Emulsion fein in diskreten Tröpfchen verteilt wird, und anschließend unter Zugabe eines Vernetzungsmittels das Kollagen in der Tröpfchengrenzfläche vernetzt wird, und schließlich die so hergestellten Kollagenpartikel durch Abtrennen der organischen Phase und durch Waschen gereinigt und isoliert werden,
**dadurch gekennzeichnet**,
daß die Kollagenpartikel nach Isolierung und Reinigung als Wirkstoffträger für Medizin, Kosmetik oder den Nahrungsmittelbereich mit wirkstoffen beladen werden.

2. Verfahren nach Anspruch 1, dadurch gekennzeichnet, daß die Partikel nach Isolierung und Reinigung mit Wirkstoff beladen werden.

3. Verfahren nach einem der Ansprüche 1 oder 2, dadurch gekennzeichnet, daß die Kollagenpartikel nach Herstellung, Abtrennung und Reinigung mit einer hohen Temperaturerniedrigungsrate (Gradienten) auf Temperaturen -50°C unter Bildung möglichst kleiner Eiskristalle schockgefroren, anschließend unter Vakuum gefriergetrocknet, sodann zur Wirkstoffbeladung in Wasser redispergiert, der Dispersion Wirkstoff, Wirkstofflösung bzw. -dispersion zugesetzt und die adsorptiv mit Wirkstoff beladenen Partikel abgetrennt und gereinigt werden.

4. Verfahren nach Anspruch 1, dadurch gekennzeichnet, daß natives Kollagen eingesetzt wird.

5. Verfahren nach Anspruch 1, dadurch gekennzeichnet, daß denaturiertes Kollagen eingesetzt wird.

6. Verfahren nach Anspruch 1, dadurch gekennzeichnet, daß ein Emulgator aus der Gruppe der Polyethylenglyklfettsäureester, Polyethylenglykolfettalkoholether, Polyethylenglykol-sorbitan-fettsäureester, der Partialfettsäureester mehrwertger Alkohole oder der Partialfettsäureester von Zucker ausgewählt ist.

7. Verfahren nach einem oder mehreren der Ansprüche 1 bis 6, dadurch gekennzeichnet, daß als Vernetzungsmittel mono- oder bifunktionelle Aldehyde oder bifunktionelle Isocyanate eingesetzt werden.

8. Verfahren nach einem oder mehreren der Ansprüche 1 bis 7, dadurch gekennzeichnet, daß zur Bildung der nicht-wassermischbaren organischen Phase natürliche, halbsynthetische oder synthetische flüssige Fette, Öle oder Wachse eingesetzt werden.

9. Verwendung der Kollagenpartikel nach den vorhergehenden Ansprüchen zur intravenösen Injektion.

## Claims

1. Process for the production of cross-linked collagen microparticles in the micrometer and nanometer range, wherein a dispersion or solution of collagen in water is distributed finely in discrete droplets in a waterimmiscible organic phase, whereby emulsifying agents are added, a water-in-oil emulsion thereby being formed, the collagen is subsequently cross-linked in the interface of the droplets by adding a cross-linking agent, and, finally, the collagen particles produced in this manner are purified and isolated by separating the organic phase and washing,
**characterized in that**,
subsequent to isolation and purification, the collagen particles are loaded as active substance carriers for use in medicine, cosmetics or in food processing.

2. Process according to claim 1, characterized in that the particles are loaded with active substance after isolation and purification.

3. Process according to one of the claims 1 or 2, characterized in that, after their production, seperation and purification, the collagen particles are shock-freezed at a high temperature reduction rate (gradient) to a temperatures of ≦ -50°C, during which process ice crystalls are formed which are as small as possible, subsequently drie-freezed under vacuum, then redispersed in water to be loaded with active substances; active substance, an active substance solution or an active substance disperion is added to the dispersion and the particles, which are loaded with active substance by adsorption, are separated and purified.

4. Process according to claim 1, characterized in that native collagen is used.

5. Process according to claim 1, characterized in that denatured collagen is used.

6. Process according to claim 1, characterized in that an emulsifying agent is selected from the group of polyethyleneglycol fatty acid esters, polyethyleneglycol fatty alcohol ether, polyethyleneglycol-sorbitane-fatty acid ester, partial fatty acid esters of polyhydric alcohols or partial fatty acid esters of sugar.

7. Process according to one or more of claims 1 to 6, characterized in that mono- or bifunctional aldehydes or bifunctional isocyanate are utilized as cross-linking agents.

8. Process according to one or more of claims 1 to 7, characterized in that, to prepare the waterimmiscible organic phase, natural, halfsynthetic or synthetic liquid fats, oils or waxes are used.

9. The use of the collagen particles according to the foregoing claims for intravenous injection.

## Revendications

1. Procédé pour la fabrication de microparticules de collagène réticulées dans la plage micrométrique et nanométrique, conformément auquel on divise finement en gouttelettes distinctes une dispersion ou une solution de collagène dans de l'eau, en recourant à l'addition d'émulsif, dans une phase organique non miscible à l'eau et sous formation d'une émulsion E/H et on réticule ensuite le collagène dans la surface limite des gouttelettes en recourant à l'addition d'un agent de réticulation et on isole et purifie finalement les particules de collagène ainsi préparées par séparation de la phase organique et par lavage, caractérisé en ce que l'on charge les particules de collagène après isolement et épuration, en tant que supports de principes actifs, de principes actifs destinés à la médecine, la cosmétique et le domaine alimentaire.

2. Procédé suivant la revendication 1, caractérisé en ce que l'on charge les particules du principe actif après leur isolement et leur épuration.

3. Procédé suivant la revendication ou 2, caractérisé en ce qu'après leur préparation, leur séparation et leur épuration, on soumet les particules de collagène à une congélation brusque (choc) à une vitesse d'abaissement de la température (gradient) jusqu'à des températures égales ou inférieures à ≦ -50°C avec formation de cristaux de glace les plus petits possibles, puis on sèche les particules de collagène par congélation sous vide, on les redisperse ensuite dans de l'eau pour le chargement en principe actif, on ajoute le principe actif, la solution de principe actif ou la dispersion de principe actif à la dispersion et on sépare et épure les particules chargées par voie adsorptive de principe actif.

4. Procédé suivant la revendication 1, caractérisé en ce que l'on utilise du collagène naturel.

5. Procédé suivant la revendication 1, caractérisé en ce que l'on utilise du collagène dénaturé.

6. Procédé suivant la revendication 1, caractérisé en ce que l'on choisit l'émulsif parmi le groupe formé par les esters d'acides gras de polyéthylèneglycol, les éthers d'alcools gras de polyéthylèneglycol, les esters d'acides gras du polyéthylèneglycol-sorbitan, les esters partiels d'acides gras d'alcools polyhydroxylés, et les esters partiels d'acides gras de sucres.

7. Procédé suivant une ou plusieurs des revendications 1 à 6, caractérisé en ce que l'on utilise des aldéhydes monofonctionnels ou bifonctionnels ou des isocyanates bifonctionnels à titre d'agents de réticulation.

8. Procédé suivant une ou plusieurs des revendications 1 à 7, caractérisé en ce que, en vue de la formation de la phase organique non miscible à l'eau, on utilise des cires, des huiles, ou des graisses liquides, naturelles, semi-synthétiques ou synthétiques.

9. Utilisation des particules de collagène suivant l'une quelconque des revendications précédentes à des fins d'injection intraveineuse.
